(19) Europäisches Patentamt
European Patent Office
Office européen des brevets

(11) Veröffentlichungsnummer: **0 215 300**
**A2**

# (12) EUROPÄISCHE PATENTANMELDUNG

(21) Anmeldenummer: **86111201.9**

(22) Anmeldetag: **13.08.86**

(51) Int. Cl.⁴: **A 61 K 31/675**

(30) Priorität: **23.08.85 DE 3530147**

(43) Veröffentlichungstag der Anmeldung: **25.03.87 Patentblatt 87/13**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Bonse, Gerhard, Dr., Wolfskaul 3, D-5000 Köln 80 (DE)**
Erfinder: **Müller, Nikolaus, Dr., Knipprather Strasse 98, D-4019 Monheim (DE)**
Erfinder: **Andrews, Peter, Dr., Gellertweg 2, D-5600 Wuppertal (DE)**

## (54) Verwendung von Diazaphosphorinen zur Bekämpfung von Endoparasiten.

(57) Die vorliegende Erfindung betrifft die Verwendung von Diazaphosphorinen Formel I

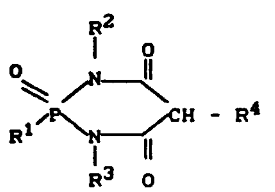

I

in welcher
$R^1$ für Aryloxy oder Arylthio steht,
$R^2$ für Wasserstoff, Alkyl, Aryl oder Aralkyl steht,
$R^3$ für Wasserstoff, Alkyl, Aryl oder Aralkyl steht,
$R^4$ für den Rest der Formel steht,

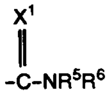

wobei
$X^1$ für O oder S steht,
$R^5$ für Wasserstoff oder Alkyl steht,
$R^6$ für gegebenenfalls substituiertes Phenyl steht,
zur Bekämpfung von Ektoparasiten in der Veterinärmedizin.

EP 0 215 300 A2

0215300

BAYER AKTIENGESELLSCHAFT
Konzernverwaltung RP
Patentabteilung

5090 Leverkusen, Bayerwerk
22. AUG. 1985
Rt/Kü-c
II

Verwendung von Diazaphosphorinen zur Bekämpfung von Endoparasiten

Die vorliegende Erfindung betrifft die Verwendung von Diazaphosphorinen zur Bekämpfung von Endoparasiten in der Veterinärmedizin.

Die Verwendung von Diazaphosphorinen zur Schädlingsbekämpfung bei Pflanzen sowie zur Bekämpfung von Ektoparasiten bei Tieren ist bereits bekannt geworden (CH-PS 543 563). Es ist jedoch nichts über ihre Eignung zur Bekämpfung von Endoparasiten, insbesondere von Helminthen, bekannt geworden.

Es wurde gefunden, daß Diazaphosphorine der Formel I

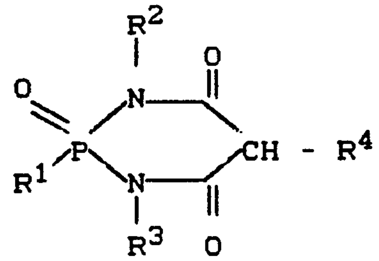

I

in welcher

$R^1$ für Aryloxy oder Arylthio steht,

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

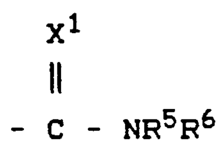

wobei

$X^1$ für O oder S steht,

$R^5$ für Wasserstoff oder Alkyl steht,

$R^6$ für gegebenenfalls substituiertes Phenyl steht,

sich hervorragend zur Bekämpfung von Endoparasiten in der Veterinärmedizin eignen.

Die Verbindungen der Formel I können in Form ihrer verschiedenen Tautomeren (Keto/Enol) sowie als Gemische dieser Tautomeren, sowie in Form ihrer Salze mit Basen vorliegen.

Die Verbindungen der Formel I sind bekannt oder lassen sich analog zu bekannten Verfahren herstellen.

Man erhält die Verbindungen der Formel I indem man z.B. Diazaphosphorine der Formel II

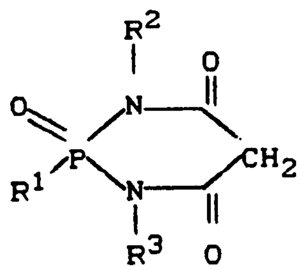

II

in welcher

$R^1$, $R^2$, $R^3$ die oben angegebene Bedeutung haben,

mit Isocyanaten der Formel III

$$R^6 - NCO(S) \qquad III$$

in welcher

$R^6$ die oben angegebene Bedeutung hat

gegebenenfalls in Gegenwart von Katalysatoren umsetzt,

oder indem man

Diazaphosphorine der Formel IV

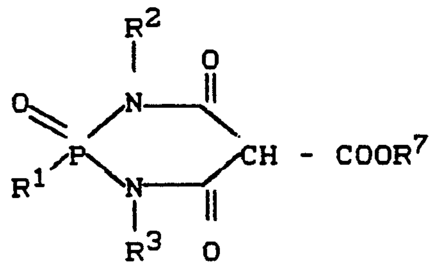

IV

in welcher

$R^1$, $R^2$, $R^3$ die oben angegebene Bedeutung haben,

$R^7$ für $C_{1-4}$-Alkyl steht

mit Aminen der Formel V

$$HNR^5R^6 \qquad V$$

in welcher

$R^5$ und $R^6$ die oben angegebene Bedeutung haben

umsetzt.

Bevorzugt sind Verbindungen der Formel I in welcher

$R^1$ für Phenoxy oder Phenylthio insbesondere Phenoxy, steht, die ein oder mehrfach gleich oder verschieden durch folgende Substituenten substituiert sein können: Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor- oder Chlorethyl; Halogenalkoxy mit vorzugsweise 1 bis 4 insbesondere 1 oder 2 Kohlenstoffatomen und vorzugsweise 1 bis 5 insbesondere 1 bis 3 Halogenatomen wobei die Halogenatome gleich oder verschieden sind und als Halogenatome vorzugsweise Fluor, Chlor, Brom insbesondere Fluor stehen wie Trifluormethoxy; Halogenalkylthio mit vorzugsweise 1 bis 4 insbesondere 1 oder 2 Kohlenstoffatomen und vorzugsweise 1 bis 5 insbesondere 1 bis 3 Halogenatomen wobei die Halogenatome gleich oder verschieden sind und als Halogenatome vorzugsweise Fluor, Chlor, Brom insbesondere Fluor stehen, wie Trifluormethylthio; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4 insbesondere 2 bis 3 Halogenatomenwobei die Halogenatome gleich oder verschieden sind und als Halogenatome vorzugweise Fluor oder Chlor insbesondere Fluor stehen wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n- Butylamino; Formyl, Carboxyl; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio die ihrerseits wieder substituiert sein können.

$R^2$ für Wasserstoff, $C_1$-$C_4$-Alkyl, Phenyl, Benzyl die gegebenenfalls substituiert sein können steht,

$R^3$ für Wasserstoff, $C_1$-$C_4$-Alkyl, Phenyl, Benzyl die gegebenenfalls substituiert sein können steht,

$X^2$ für O steht,

$R^5$ für Wasserstoff oder $C_{1-4}$-Alkyl steht,

$R^6$ für Phenyl steht, das gegebenenfalls einen oder mehrere der folgenden Substituenten trägt: Alkyl mit vorzugsweise 1 bis 4, insbesondere 1 oder 2 Kohlenstoffatomen, wie Methyl, Ethyl, n- und i-Propyl und n-, i- und t-Butyl; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom insbesondere Fluor stehen, wie Trifluormethyl; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-, Butylamino; Formyl; Carboxyl; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Arylsulfonyl mit vorzugsweise 6 oder 10 Arylkohlenstoffatomen, wie Phenylsulfonyl; Phenyl, Naphthyl, die ihrerseits wieder substituiert sein können.

Besonders bevorzugt sind Verbindungen der Formel I

in welcher

$R^1$ für Phenoxy steht das gegebenenfalls einen oder mehrere der folgenden Substituenten trägt:

Halogen insbesondere Fluor, Chlor, Brom, $NO_2$, $C_1$-$C_4$-Alkoxy insbesondere Methoxy, Ethoxy, $C_1$-$C_4$-Alkyl insbesondere Methyl, Ethyl, Propyl, $C_1$-$C_4$-Akylthio insbesondere Methylthio, Ethylthio, $C_1$-$C_4$-Halogenalkyl insbesondere Trifluormethyl, $C_1$-$C_4$-Halogenalkoxy insbesondere Trifluormethoxy, $C_1$-$C_4$-Halogenalkylthio insbesondere Trifluormethylthio. Bevorzugt stehen dabei die Substituenten in 3- und 4-Stellung des Phenylringes.

$R^2$ für $C_1$-$C_4$-Alkyl, Phenyl, Benzyl steht,

$R^3$ für $C_1$-$C_4$-Alkyl, Phenyl, Benzyl steht,

$X^1$ für O steht,

$R^5$ für Wasserstoff steht,

$R^6$ für Phenyl steht, das gegebenenfalls substituiert ist durch Nitro, Halogen, insbesondere Fluor, Chlor, Brom, $C_1$-$C_4$-Alkyl insbesondere Methyl oder Ethyl, $C_1$-$C_4$-Halogenalkyl insbesondere Trifluormethyl.

Ganz besonders bevorzugt sind Verbindungen der Formel I in welcher

$R^1$ für Phenoxy steht, das gegebenenfalls in 3 oder 4-Stellung substituiert ist durch Halogen, insbesondere Chlor, $NO_2$, $C_1$-$C_4$-Alkyl, insbesondere Methyl, $C_1$-$C_4$-Alkoxy, insbesondere Methoxy,

$R^2$ für $C_1$-$C_4$-Alkyl insbesondere Methyl oder Ethyl, Phenyl steht,

$R^3$ für $C_1$-$C_4$-Alkyl insbesondere Methyl, Ethyl, Propyl, Benzyl steht,

$X^2$ für O steht,

$R^5$ für Wasserstoff steht,

$R^6$ für Phenyl steht, gegebenenfalls durch $NO_2$, Chlor oder Methyl substituiert ist.

Im einzelnen seien folgende Verbindungen der Formel I genannt:

$R^4 = CX^1NR^5R^6$

| $R^1$ | $R^2$ | $R^3$ | $X^1$ | $R^5$ | $R^6$ |
|---|---|---|---|---|---|
| 4-Cl-$C_6H_4O$ | $C_6H_5$ | $-CH_3$ | O | H | 3-Cl, 4-$CF_3$-$C_6H_3$ |
| 4-$NO_2$-$C_6H_4O$ | $-C_6H_5$ | $-CH_3$ | S | $C_2H_5$ | 3,4-$Cl_2$-$C_6H_3$ |
| 3,4-$Cl_2$-$C_6H_3O$ | $-C_6H_5$ | $-CH_3$ | O | $CH_3$ | 3-Cl, 4-$CF_3$-$C_6H_3$ |
| 4-Cl-$C_6H_4O$ | $-CH_3$ | $-CH_3$ | O | H | 3-Cl, 4-$CF_3$-$C_6H_3$ |
| 4-$NO_2$-$C_6H_4O$ | $-CH_3$ | $-CH_3$ | O | $CH_3$ | 3,4-$Cl_2$-$C_6H_3$ |

Als Basen mit denen die Verbindungen der Formel I Salze bilden können seien genannt: Alkali- und Erdalkalihydroxide, Ammoniak, primäre, sekundäre und tertiäre Amine. Besonders bevorzugt seien folgende Basen genannt: Triethylamin, Isopropylamin, t-Butylamin, Pyridin, α-Picolin, Trimethylamin, Diisopropylamin, Morpholin, Pyrrolidin, Hexamethylenimin.

Die erfindungsgemäßen Wirkstoffe sind breit wirksam gegen Endoparasiten. Sie wirken vor allem gegen Trematoden und Nematoden, insbesondere Leberegel und Magen- und Darmnematoden der Wiederkäuer. Darüber hinaus wirken sie auch gegen solche Magen- und Darmnematoden, die gegen die gebräuchlichen Benzimidazol-Anthelmintika resistent und damit nicht mehr ausreichend therapierbar sind.

Die Wirkung wurde im Tierversuch nach oraler, parenteraler und dermaler Applikation bei stark mit Parasiten befallenen Versuchstieren geprüft. Die angewendeten Dosierungen wurden sehr gut von den Versuchstieren vertragen.

Die erfindungsgemäßen Wirkstoffe können als Anthlemintika verwendet werden.

Die erfindungsgemäßen Wirkstoffe können zusammen mit anderen üblichen Anthelmintika verabreicht werden.

Die erfindungsgemäßen Wirkstoffe können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine

orale Verabreichung in Betracht kommenden Tabletten und dergleichen mit Süßstoffzusatz und ähnlichen versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um den obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:
Wasser, nicht toxische organische Lösungsmittel, wie Paraffine (z.B. Erdölfraktionen), pflanzliche Öle (z.B. Erdnuß-(Sesamöl), Alkohole (z.B. Ethylalkohol, Glycerin), Glykole (z.B. Propylenglykol, Polyethylenglykol) und Wasser; feste Trägerstoffe, wie z.B. natürliche Gesteinsmehle (z.B. Kaoline, Tonerden, Talkum, Kreide) synthetische Gesteinsmehle (z.B. hochdisperse Kieselsäure, Silikate), Zucker (z.B. Rohr-, Milch- und Traubenzucker); Emulgiermittel, wie nicht ionogene und anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze wie Natriumcitrat, Calciumcarbonat und Dicalciumphosphat, zusammen mit verschiedenen Zuschlagstoffen, wie Stärke, vorzugsweise Kartoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für die orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen Dragees, Ampullen usw. auch in Form von Dosierungseinheiten sein, wobei jede Dosierungseinheit so angepaßt ist, daß sie eine einzelne Dosis des aktiven Bestandteils liefert.

Die erfindungsgemäßen Wirkstoffe können in den Formulierungen auch in Mischungen mit anderen in der Veterinär- und/oder Humanmedizin zur Behandlung von Infektionen und/oder Erkrankungen benutzten bekannten Wirkstoffen vorliegen, insbesondere L-2,3,5,6-Tetrahydro-6-phenylimidazothiazol, Benzimidazolcarbamaten, Praziquantel, Febantel.

0215300

Die Wirkstoffe können in üblicher Weise angewendet werden. Die Applikation erfolgt vorzugsweise oral, eine parenterale, insbesondere subkutane, aber auch eine dermale Applikationen (pour-on, spot-on) sind möglich.

Im allgemeinen hat es sich als vorteilhaft erwiesen, Mengen von etwa 1 bis etwa 100 mg der Wirkstoffe je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Veterinärmedizin ist der gleiche Dosierungsspielraum vorgesehen. Sinngemäß gelten auch die weiteren obigen Ausführungen.

## Beispiel A

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wurde auf eine Replica-Platte gegeben. Dazu wurden 2 ml einer E.coli-Suspension gegeben, zu der man 10-20 weibliche Tiere oder Larven von Caenorhabditis elegans in 0,5 ml sterile M9 Pufferlösung gegeben hat. Die E.coli-Suspension wurde hergestellt indem man 300 ml einer Übernachtkultur eines Uracil-bedürftigen E.coli-Stammes mit 1,8 l steriler M9 Pufferlösung versetzte.

Der Versuchsansatz wurde 7 Tage bei 22°C inkubiert und danach ausgewertet. Es wurde bewertet inwieweit der Wirkstoff die Vermehrung beeinträchtigt und die Konzentration angegeben bei der die Vermehrung verhindert wird. Dabei wurden folgende Ergebnisse erhalten:

0215300

In vitro Nematodentest

Caenorhabditis elegans

| Wirkstoff Beispiel Nr. | Minimale effektive Dosis mg/l |
|---|---|
| 1 | 10 |
| 2 | 100 |
| 3 | 100 |
| 6 | 1 |
| 8 | 10 |
| 10 | 1 |
| 11 | 1 |
| 12 | 100 |
| 13 | 10 |
| 14 | 100 |
| 15 | 10 |
| 17 | 10 |
| 18 | 10 |
| 20 | 10 |
| 22 | 10 |
| 27 | 10 |
| 28 | 100 |
| 34 | 100 |
| 35 | 10 |
| 36 | 10 |
| 37 | 10 |

Beispiel B

In vivo Nematodentest

Heterakis spumora

Experimentell mit Heterakis spumora infizierte Mäuse werden 32 Tage nach der Infektion an vier aufeinanderfolgenden Tagen oral mittels Schlundsonde behandelt. Die Tiere werden 39 Tage nach der Infektion getötet und die Zahl der Parasiten bestimmt. Es wird die Wirkstoffkonzentration angegeben bei der mindestens 95 % der Parasiten abgetötet wurden (minimale effektive Dosis):

| Wirkstoff Beispiel Nr. | minimale effektive Dosis mg/kg |
|---|---|
| 22 | 250 |

Beispiel C

In vivo Nematodentest

Hymenolepis nana

Experimeltell mit Hymenolepis nana infizierte Mäuse werden 10 Tage nach der Infektion an vier aufeinanderfolgenden Tagen oral mittels Schlundsonde behandelt. Die Tiere werden 17 Tage nach Infektion getötet und die Zahl der Parasiten bestimmt. Es wird die Wirkstoffkonzentration angegeben, bei der mindestens 95 % der Parasiten abgetötet wurden (minimale effektive Dosis):

| Wirkstoff Beispiel Nr. | minimale effektive Dosis mg/kg |
|---|---|
| 22 | 250 |

Beispiel D

In vivo Nematodentest

Strongyloides ratti

Experimentell mit Strongyloides ratti infizierte Ratten werden 7 Tage nach der Infektion an drei aufeinanderfolgenden Tagen oral mittels Schlundsonde behandelt. Die Tiere werden 13 Tage nach der Infektion getötet und die Zahl der Parasiten bestimmt. Es wird die Wirkstoffkonzentration angegeben, bei der mindestens 95 % der Parasiten abgetötet wurden (minimale effektive Dosis):

| Wirkstoff Beispiel Nr. | minimale effektive Dosis mg/kg |
|---|---|
| 8 | 25 |
| 11 | 10 |
| 20 | 250 |

## Beispiele

a) <u>Allgemeine Vorschrift zur Herstellung der erfindungsgemäßen 5-Carbamoyl substituierten 1,3,2-Diazaphosphorine gemäß Verfahren a)</u>

Je 0,03 Mol des 1,3,2-Diazaphosphorins und des Isocyanats werden in 120 ml trockenem THF gelöst vorgelegt und bei Raumtemperatur innerhalb von 30 min. mit einer Lösung von 0,03 Mol (4,56 g) "DBU" in 30 ml THF versetzt. Die anfänglich hellbraune Lösung verdunkelt sich und es tritt eine leichte Wärmeentwicklung auf, so daß die Innentemperatur durch Wasserkühlung unter 25°C gehalten werden muß. Anschließend wird bei Raumtemperatur bis zum vollständigen Umsatz nachgerührt (ca. 4-5 h, DC-Probe!). Dann wird der gesamte Ansatz eingedampft, in 100 ml Methylenchlorid aufgenommen und mit 100 ml verdünnter Salzsäure (10 %ig) verrührt. Die organische Phase wird abgetrennt, mit wäßriger Natriumhydrogencarbonatlösung neutral gewaschen und über Natriumsulfat getrocknet. Nach dem Entfernen des Lösungsmittels am Rotationsverdampfer wird der Rückstand aus Ligroin oder Isopropanol umkristallisiert oder chromatographiert.

Analog werden die Wirkstoffe der folgenden Beispiele erhalten:

Tabelle 2

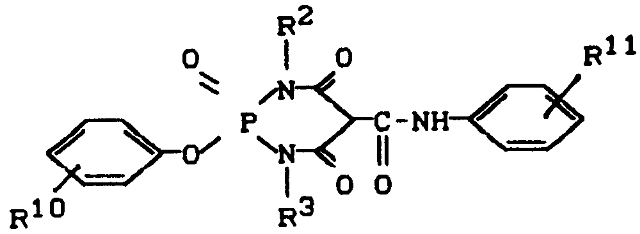

Tabelle 2

| Bsp. Nr. | $R^{10}$ | $R^2=R^3$ | $R^{11}$ | Fp/°C |
|---|---|---|---|---|
| 1 | 4-Cl | $CH_3$ | 3-$CF_3$ | 104-106 |
| 2 | 3-Cl | i-$C_3H_7$ | 3-Cl | Öl |
| 3 | 3-Cl | i-$C_3H_7$ | 3-$CF_3$ | Öl |
| 4 | 3-$CH_3$ | i-$C_3H_7$ | 3-Cl | Öl |
| 5 | 3-$CH_3$ | i-$C_3H_7$ | 3-$CF_3$ | Öl |
| 6 | 4-Cl | $CH_3$ | 4-$CF_3$ | 152-154 |
| 7 | 4-$NO_2$ | $CH_3$ | 3-Cl | 205 |
| 8 | H | $CH_3$ | 4-$CF_3$ | 122-124 |
| 9 | H | $CH_3$ | 3-Cl, 4-F | 115-117 |
| 10 | 4-Cl | $CH_3$ | 4-Cl | 165 |
| 11 | 3-Cl | $CH_3$ | 3-Cl | 91-93 |
| 12 | 4-Cl | $CH_3$ | 3-Cl | 84-86 |
| 13 | 4-Cl | $C_6H_5$ | 3,4-$Cl_2$ | 188-190 |
| 14 | H | $C_6H_5$ | 3,4-$Cl_2$ | 179-182 |

0215300

| Bsp. Nr. | $R^{10}$ | $R^2$ | $R^3$ | $R^{11}$ | Fp/°C |
|---|---|---|---|---|---|
| 16 | 3-Cl | $i\text{-}C_3H_7$ | $CH_3$ | 3,4-$Cl_2$ | Öl |
| 17 | 3-Cl | $i\text{-}C_3H_7$ | $CH_3$ | 4-$CF_3$ | Öl |
| 18 | 3-Cl | $i\text{-}C_3H_7$ | $CH_3$ | 3-Cl | 89-92 |
| 19 | 3-Cl | $C_2H_5$ | $CH_3$ | 3,4-$Cl_2$ | Öl |
| 20 | 3-Cl | $C_2H_5$ | $CH_3$ | 3-$CF_3$ | Öl |
| 21 | 3-Cl | $C_2H_5$ | $CH_3$ | 3-Cl | Öl |
| 22 | 3-Cl | $i\text{-}C_3H_7$ | $CH_3$ | 3-$CF_3$ | Öl |
| 23 | 3-Cl | $i\text{-}C_3H_7$ | $-CH_2-C_6H_5$ | 3-Cl | Öl |
| 24 | 3-Cl | $i\text{-}C_3H_7$ | $-CH_2-C_6H_5$ | 3-$CF_3$ | Öl |
| 25 | 3-$CH_3$ | $i\text{-}C_3H_7$ | $-CH_2-C_6H_5$ | 3-Cl | Öl |
| 26 | 3-$CH_3$ | $i\text{-}C_3H_7$ | $-CH_2-C_6H_5$ | 3-$CF_3$ | Öl |
| 27 | 3-$CH_3$ | $i\text{-}C_3H_7$ | $CH_3$ | 3-$CF_3$ | Öl |
| 28 | 3-$CH_3$ | $i\text{-}C_3H_7$ | $CH_3$ | 3-Cl | Öl |
| 29 | H | $CH_3$ | $C_6H_5$ | 3,4-$Cl_2$ | 164-166 |
| 30 | 3-$CH_3$ | $CH_3$ | $CH_2-C_6H_5$ | 3-Cl | Öl |
| 31 | 3-$CH_3$ | $CH_3$ | $CH_2-C_6H_5$ | 3-$CF_3$ | Öl |
| 32 | 3-Cl | $CH_3$ | $CH_2-C_6H_5$ | 3-Cl | Öl |
| 33 | 3-Cl | $CH_3$ | $CH_2-C_6H_5$ | 3-$CF_3$ | Öl |
| 34 | 3-$CH_3$ | $CH_3$ | H | 3-$CF_3$ | amorph |
| 35 | 3-$CH_3$ | $CH_3$ | $i\text{-}C_3H_7$ | 3,4-$Cl_2$ | amorph |
| 36 | 3-Cl | $CH_3$ | $CH_3$ | 3-$CF_3$ | 89-91 |
| 37 | 4-Cl | $C_6H_5$ | $CH_3$ | 3,4-$Cl_2$ | 142-143° |
| 38 | H | 4-$C_6H_4Cl$ | $CH_3$ | 3,4-$Cl_2$ | 126-130° |

## Patentansprüche

1. Verwendung von Diazaphosphorinen der Formel I

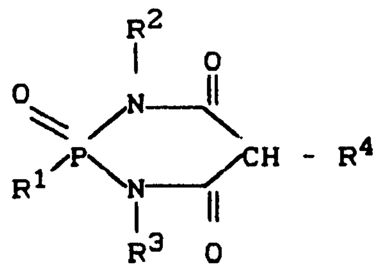

I

in welcher

$R^1$ für Aryloxy oder Arylthio steht,

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

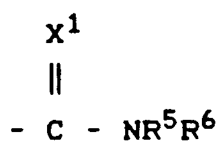

wobei

$X^1$ für O oder S steht,

$R^5$ für Wasserstoff oder Alkyl steht,

$R^6$ für gegebenenfalls substituiertes Phenyl steht,

zur Bekämpfung von Endoparasiten in der Veterinärmedizin.

2. Mittel zur Bekämpfung von Endoparasiten, gekennzeichnet durch einen Gehalt an mindestens einem Diazaphosphorin der Formel I gemäß Anspruch 1.

3. Verwendung von Diazaphosphorinen der Formel I gemäß Anspruch 1 zur Bekämpfung von Endoparasiten.

4. Verfahren zur Herstellung von endoparatiziden Mitteln, dadurch gekennzeichnet, daß man Diazaphosphorine der Formel I gemäß Anspruch 1 verwendet.

5. Verwendung von Diazaphosphorinen der Formel I

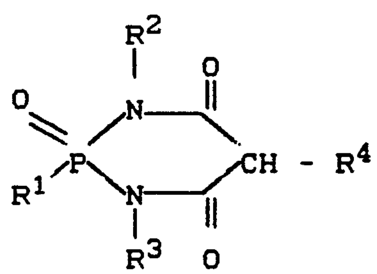

I

in welcher

$R^1$ für Aryloxy oder Arylthio steht,

$R^2$ für Wasserstoff, Alkyl, Aryl oder Aralkyl steht,

0215300

$R^3$ für Wasserstoff, Alkyl, Aryl oder Aralkyl steht,

$R^4$ für den Rest der Formel steht,

$$-\overset{\displaystyle X^1}{\overset{\|}{C}}-NR^5R^6$$

wobei

$X^1$ für O oder S steht,

$R^5$ für Wasserstoff oder Alkyl steht,

$R^6$ für gegebenenfalls substituiertes Phenyl steht,

zur Herstellung von endoparatiziden Mitteln.